# EUROPEAN PATENT SPECIFICATION

(11) **EP 4 256 322 B1**
(45) Date of publication and mention of the grant of the patent: **01.10.2025**
(21) Application number: 21819462.9
(22) Date of filing: 29.11.2021
(51) Int. Cl.: G01N 33/00

(54) **MODULAR AIR QUALITY SENSING SYSTEM**
MODULARES LUFTQUALITÄTSMESSSYSTEM
SYSTÈME DE DÉTECTION DE LA QUALITÉ DE L'AIR MODULAIRE

(30) Priority: 01.12.2020 US 202063119828 P; 04.12.2020 EP 20211817
(43) Date of publication of application: 11.10.2023
(73) Proprietor: Signify Holding B.V., 5656 AE Eindhoven (NL)
(72) Inventor: TAN, Peng Seng, 5656 AE Eindhoven (NL); SHEWALKAR, Gaurav Satish, 5656 AE Eindhoven (NL)
(74) Representative: Verweij, Petronella Daniëlle
(86) International application number: PCT/EP2021/083396
(87) International publication number: WO 2022/117511

(56) References cited:
- WO-A2-2011/090763
- US-A1- 2015 259 078
- US-A1- 2016 178 586
- US-A1- 2020 051 416
- US-A1- 2020 363 043
- KIM JUNG-YOON ET AL: "ISSAQ: An Integrated Sensing Systems for Real-Time Indoor Air Quality Monitoring", IEEE SENSORS JOURNAL, IEEE SERVICE CENTER, NEW YORK, NY, US, vol. 14, no. 12, 1 December 2014 (2014-12-01), pages 4230 - 4244, XP011562497, ISSN: 1530-437X, [retrieved on 20141022], DOI: 10.1109/JSEN.2014.2359832
- PIMORONI: "Enviro for Raspberry Pi - Monitor your world! - Pimoroni Store", 3 December 2019 (2019-12-03), XP055805523, Retrieved from the Internet <URL:https://web.archive.org/web/20191203021713if_/https://shop.pimoroni.com/web/20191203021713if_/https://shop.pimoroni.com/web/20191203021713if_/https://shop.pimoroni.com/web/20191203021713if_/https://shop.pimoroni.com/web/20191203021713if_/https://shop.pimoroni.com/web/20191203021713if_/https://shop.p> [retrieved on 20210518]

## Description

### FIELD OF THE DISCLOSURE

The present disclosure is directed generally to systems and methods for dynamically configuring air quality sensors. More specifically, the present disclosure is directed to systems and methods for dynamically configuring the types of air quality sensing capabilities that are provided in lighting products and accessories.

### BACKGROUND

Conventional indoor air quality sensors are provided in desktop models, wall-mounted models, or devices that are plugged into wall receptacles. An example of conventional sensing systems can be found in US2016/178586A1, which describes an electrical outlet enclosures with built in air quality sensors. Aside from occupying space in the indoor space, these sensors provide a fixed, limited number of capabilities. For example, some sensors detect humidity, temperature, and carbon dioxide levels but not allow for new types of air quality sensors to be added into them for monitoring different components of the indoor air quality. Other sensors detect a large number of components of the indoor air quality but do not allow for types of air quality sensors to be removed. These current devices do not allow for the dynamic configuration of different types of air quality sensors such that different types of air quality sensors can be swapped in or out.

Accordingly, there is a need in the art for improved systems and methods for dynamically configuring an air quality sensor module with different capabilities.

### SUMMARY OF THE INVENTION

The present disclosure is directed to systems according to the appended claims.

In various implementations, the microcontroller described herein may take any suitable form, such as, one or more processors or microcontrollers, circuitry, one or more controllers, a field programmable gate array (FGPA), or an application-specific integrated circuit (ASIC) configured to execute software instructions. Memory associated with the microcontroller may take any suitable form or forms, including a volatile memory, such as random-access memory (RAM), static random-access memory (SRAM), or dynamic random-access memory (DRAM), or non-volatile memory such as electrically erasable programmable read-only memory (EEPROM), flash memory, or other non-transitory machine-readable storage media. The term "non-transitory" means excluding transitory signals but does not further limit the forms of possible storage. In some implementations, the storage media may be encoded with one or more programs that, when executed on one or more processors and/or controllers, perform at least some of the functions discussed herein. It will be apparent that, in embodiments where the microcontroller implements one or more of the functions described herein in hardware, the software described as corresponding to such functionality in other embodiments may be omitted. Various storage media may be fixed within a processor or may be transportable, such that the one or more programs stored thereon can be loaded into the processor so as to implement various aspects as discussed herein. Data and software, such as the algorithms or software necessary to analyze the data collected by the sensors, an operating system, firmware, or other application, may be installed in the memory.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the present disclosure.
FIG. 1A is an example mountable electrical outlet enclosure having a removable panel to accommodate a modular air quality sensor in accordance with aspects of the present disclosure;
FIG. 1B is an example mountable switch having a removable panel to accommodate a modular air quality sensor in accordance with aspects of the present disclosure;
FIG. 1C is an example mountable sensor and switch having a removable panel to accommodate a modular air quality sensor in accordance with aspects of the present disclosure;
FIG. 2 is an example schematic depiction of a frontend of a modular air quality sensor in accordance with aspects of the present disclosure;
FIG. 3 is an example schematic depiction of a backend of the modular air quality sensor of FIG. 2 in accordance with aspects of the present disclosure;
FIG. 4 is an example flowchart showing a method for dynamically configuring a modular air quality sensing device in accordance with aspects of the present disclosure; and
FIG. 5 is an example air quality sensing system in accordance with aspects of the present disclosure.

### DETAILED DESCRIPTION OF EMBODIMENTS

The present disclosure describes various embodiments of systems and methods for dynamically configuring an air quality sensing device such that different sensing capabilities can be added or removed without having to use multiple sensing products. Applicant has recognized and appreciated that it would be beneficial to integrate a dynamically configurable air quality sensor component within wall stations or wall receptacles that are already part of a commercial or residential structure. Existing connected devices, such as, fixed or portable, battery powered wall stations or wall receptacles, can be used as a backbone for the additional modular air quality sensing functionalities described herein.

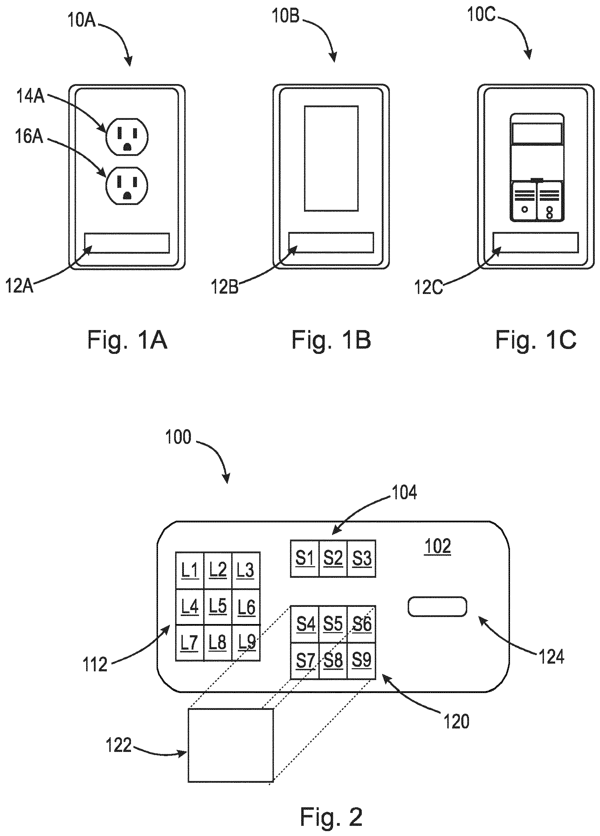

The following should be appreciated in view of FIGS. 1A, 1B, 1C, 2, and 3. The modular air quality sensor described below can be installed within an electrical outlet or a switch enclosure. The enclosures contemplated herein are configured to allow airflow to permit the integrated one or more sensors to be exposed to the air of the ambient environment of the sensor. As shown in FIG. 1A, an example mountable electrical outlet enclosure 10A having a removable panel 12A is depicted. Removable panel 12A is configured to accommodate a modular air quality sensor as described below. An advantage of such an embodiment is that, when integrated, the modular air quality sensor does not occupy either of the receptacles 14A, 16A so that the receptacles 14A, 16A are available for other uses aside from sensing air quality parameters of the air surrounding the enclosure 10A. Integrating the modular air quality sensor in this way also does not prevent either of the receptacles 14A, 16A from being used. Another advantage of such an embodiment is that, when integrated, the modular air quality sensor does not occupy additional space than already used by the enclosure 10A. FIG. 1B shows an example mountable switch enclosure 10B having a removable panel 12B to accommodate a modular air quality sensor as described below. The switch enclosure 10B features the same advantages as discussed above with respect to the electrical outlet enclosure 10A. An example mountable sensor and switch enclosure 10C having a removable panel 12C to accommodate a modular air quality sensor as described below is shown in FIG. 1C. The sensor and switch combination enclosure 10C features the same advantages as discussed above with respect to the electrical outlet enclosure 10A. The modular air quality sensor described herein is configured to replace the space that is taken by the snap on/off removable panels 12A, 12B, and 12C. Although the panels 12A, 12B, and 12C are shown as rectangular panels, it should be appreciated that the panels can take any suitable shape. Additionally, it should be appreciated that the panels can be removable by any suitable means.

The enclosures contemplated herein are not limited to the embodiments depicted in FIGS. 1A, 1B, and 1C.

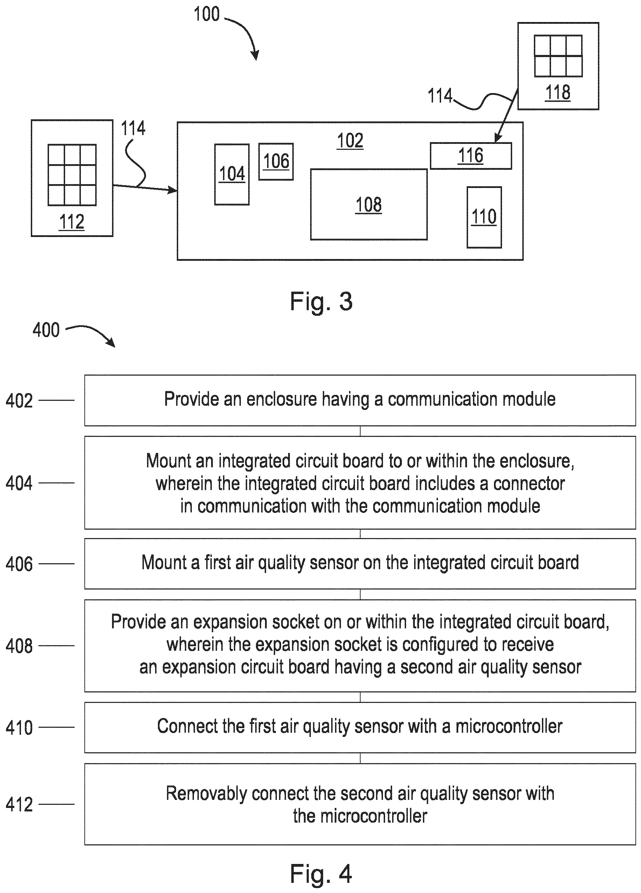

As shown in FIG. 2, a frontend of an example modular air quality sensor 100 is depicted. A backend of the example modular air quality sensor 100 is shown in FIG. 3. The modular air quality sensor 100 includes an integrated circuit board 102, at least one onboard air quality sensor 104, and an onboard microcontroller 106 including a processor, a memory, and input and output peripherals. The onboard microcontroller 106 is configured to communicate with all of the installed air quality sensors via a I-squared-C (I2C) interface. The onboard microcontroller 106 is configured to receive sensor data captured by the at least one onboard air quality sensor and any additional sensors discussed herein via the input peripherals and determine via the processor whether the sensor data meets or exceeds one or more threshold levels pertaining to the air quality. The sensor data as well as the threshold levels can be stored in the memory of the microcontroller 106. The onboard microcontroller is also configured to receive additional sensor information, such as, data indicating a status of the sensor (e.g., whether it is active or inactive), a health status of the sensor, a power level or battery level of the sensor, etc. Such additional information can be stored in the memory. The onboard microcontroller 106 can also be configured to query the sensors to determine if they are active, inactive, and/or functioning properly.

In embodiments, the at least one onboard air quality sensor 104 of the modular air quality sensor 100 can include a trio of primary sensors, such as, a carbon dioxide sensor S1, a temperature sensor S2, and a humidity sensor S3 mounted on the integrated circuit board 102. However, it should be appreciated that any types of sensors can be included and the trio is not limited to a carbon dioxide sensor, a temperature sensor, and a humidity sensor. It should also be appreciated that the at least one onboard air quality sensor can include only a duo of primary sensors or more than three primary sensors. It should further be appreciated that it is envisioned that the integrated circuit board is configured to integrate future types and/or forms of air quality sensors that have improved capabilities. The modular air quality sensor 100 also includes a connector 108 with I-squared-C (I2C) or universal asynchronous receiver-transmitter (UART) communication pin interfaces that can connect to a Wi-Fi/BLE communication module within the wall station or receptacle enclosure to disseminate sensor status information via the wireless radio of the enclosure to one or more remote entities, such as, any building management systems or any suitable cloud-based services. The 5V power supply and grounding are also provided by the same connector 108.

In embodiments, the modular air quality sensor 100 also includes an onboard Wi-Fi/BLE combination radio module 110 for wireless communication to any suitable remote entities, such as, any building management systems or any suitable cloud-based services. Thus, in embodiments the onboard microcontroller 106 is also configured to output data to the radio module 110 via the output peripherals.

In embodiments, the modular air quality sensor 100 includes an array of light emitting elements 112, such as, light-emitting diodes or LEDs, connected to the integrated circuit board 102. In embodiments, the array of light emitting elements 112 can be connected to the integrated circuit board 102 via any suitable connecting means 114, such as, a flexible flat cable. In embodiments, the array of light emitting elements 112 comprises at least one element for each primary sensor preconfigured on the integrated circuit board 102 and each additional sensor connected through the expansion socket discussed further below. Each of the light emitting elements indicates the status of the air quality components that are connected to the integrated circuit board 102. In embodiments, the array comprises nine LEDs arranged in a 3x3 grid as depicted L1, L2, L3, L4, L5, L6, L7, L8, and L9. However, it should be appreciated that any arrangement is contemplated. The LEDs can be driven by the onboard microcontroller 106 using the microcontroller's general purpose input/output (GPIO) pins. The light emitting elements 112 can be configured to present various colors and/or lighting patterns.

The modular air quality sensor 100 including the integrated circuit board 102, the at least one onboard air quality sensor 104 (e.g., at least one of S1, S2, and S3), the onboard microcontroller 106, communication means, and LED status array can be packaged in a casing or housing/enclosure such that the onboard sensors are mounted on the frontend of the casing along with the LED status array. Power to the modular air quality sensor 100 (e.g., 5V) is provided from the enclosure.

The modular air quality sensor 100 also includes a user input 124 on the frontend of the casing to turn on and off power to the integrated circuit board 102. The user input 124 may be, for example, a button, a touch screen, or switch.

The modular air quality sensor 100 further includes an expansion socket 116 mounted on, arranged within, or otherwise connected to the integrated circuit board 102. In embodiments, the expansion socket 116 can be connected to the integrated circuit board 102 via any suitable connecting means 114, such as, a flexible flat cable. The expansion socket 116 is configured to receive an expansion integrated circuit board 118 having at least one additional air quality sensor 120. As shown in FIG. 2, the additional air quality sensors can include any combination of additional sensors S4, S5, S6, S7, S8, and S9, for example, a carbon dioxide sensor, a carbon monoxide sensor, a total volatile organic compound (VOC) sensor, an ammonium detector, a dust particle sensor (e.g., a fine particulate matter sensor PM2.5 or a coarse particulate matter sensor PM10), a nitrogen oxide sensor, an ionization smoke detector, a photoelectric smoke detector, a radon sensor or other poisonous gas detection sensors, among others. It should also be appreciated that it is envisioned that the expansion socket is configured to receive or accommodate future types and/or forms of air quality sensors that have improved capabilities.

In the embodiment depicted, three of the light emitting elements 112, such as, L1, L2, and L3 indicate the status of the air quality sensors S1, S2, and S3. The additional light emitting elements 112, such as, L4, L5, L6, L7, L8, and L9 indicate the status of additional air quality sensors S4, S5, S6, S7, S8, and S9. It should be appreciated that all of the additional light emitting elements L4, L5, L6, L7, L8, and L9 need not be illuminated at the same time if not all of the additional air quality sensors S4, S5, S6, S7, S8, and S9 are installed and active. Additionally, if a user wants to supplement sensors S1, S2, and S3 with only a single additional air quality sensor, then the integrated circuit board 118 would include only a single additional air quality sensor 120 and only L4, L5, L6, L7, L8, or L9 would be used to indicate the status of the additional air quality sensor 120.

The expansion socket 116 also includes power and I2C interface pins to allow for connection to the expansion integrated circuit board 118 including the one or more additional air quality sensors 120, S4, S5, S6, S7, S8, and S9.

In embodiments, the modular air quality sensor 100 includes a snap on/off or otherwise removable panel 122 at the topside shown in FIG. 2 that can be removed to allow the expansion module, including the expansion integrated circuit board 118 and at least one additional air quality sensor 120, to be inserted into the expansion socket 116 of the integrated circuit board.

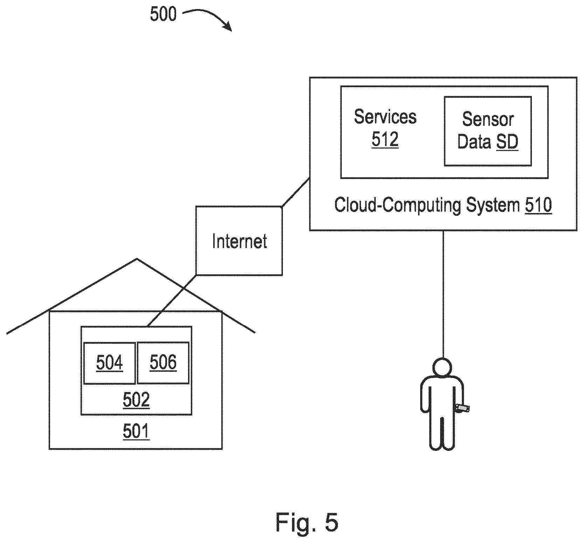

Referring to FIG. 4, an example method for dynamically configuring an air quality sensing device is shown. Unless explicitly stated, it should be appreciated that the steps are recited without regard to a particular order or sequence. In step 402, an enclosure (e.g., a mountable electrical outlet enclosure 10A, a mountable switch enclosure 10B, a mountable sensor and switch enclosure 10C, or other suitable enclosure) is provided where the enclosure includes a communication module 504 (shown in FIG. 5). It should be appreciated that the enclosure is not limited to the embodiments shown in FIGS. 1A, 1B, and 1C. The embodiment of the enclosure as well as the placement of the enclosure (e.g., on a wall, on a ceiling, or underground, etc.) largely depends on the types of sensors selected. For example, an air quality sensing device that includes a radon sensor would be placed within the breathing zone, 2-6 feet above the floor and away from drafts, exterior walls, sumps, drains, windows or door. By way of another example, an air quality sensing device that includes an ozone detector, that is configured to detect harmful levels of ozone emitted by printing devices, can be placed proximate to the device to be monitored. A carbon monoxide detector can be placed proximate to a gas stove, a chimney, a fireplace, or a furnace to be monitored. Depending on the density of one or more gases to be detected it may be more beneficial to place the enclosure and sensor on the ceiling versus the wall and vice versa or elsewhere.

In step 404, an integrated circuit board (e.g., board 102) is mounted to or within or otherwise connected to the enclosure. The integrated circuit board includes a connector (e.g., connector 108) in communication with the communication module 504 of the enclosure.

In step 406, at least one air quality sensor is mounted on the integrated circuit board. In embodiments, the at least one air quality sensor is at least one of the onboard primary sensors discussed herein (e.g., sensors S1, S2, or S3).

In step 408, an expansion socket is provided on or within or otherwise connected to the integrated circuit board. The expansion socket is configured to receive an expansion integrated circuit board having at least one additional air quality sensor. In embodiments, the at least one additional air quality sensor is at least one of the additional sensors discussed herein (e.g., sensors S4, S5, S6, S7, S8, and S9). In embodiments, at least one additional air quality sensor is removable from the expansion integrated circuit board so that a user can customize the types and number of additional sensors. It should be appreciated that the at least one additional air quality sensor can be added or removed individually or in a group-wise manner. Thus, combinations of additional sensors can be installed via the expansion socket.

In step 410, at least one onboard air quality sensor is connected with an onboard microcontroller.

In step 412, the at least one additional air quality sensor is removably connected with the onboard microcontroller via the expansion socket.

Advantageously, the modular air quality sensing systems and methods allow a consumer to dynamically customize the air sensing capabilities of the product while providing a less intrusive look of the environment.

FIG. 5 shows an example air quality sensing system 500 within which one or more of the modular air quality sensors described herein can be implemented. The system 500 includes a structure 501 which can include a house, office building, warehouse, etc. It should be appreciated that the structure 501 can include an entire structure or part of the structure, such as, an apartment or an office suite within a building. The system 500 also includes a plurality of intelligent, multi-sensing, network-connected devices, that can integrate seamlessly with each other and/or with a central server or a cloud-computing system to provide any of a number of useful smart-home objectives. For example, the system can include one or more intelligent, multi-sensing, network-connected thermostats, one or more intelligent, multi-sensing, network-connected wall switches, one or more intelligent, multi-sensing, network-connected wall plug interfaces, one or more intelligent, multi-sensing, network-connected luminaires, and one or more intelligent, multi-sensing, network-connected appliances, such as, ceiling fans and air conditioners. The modular air quality sensors described herein can be integrated in any of these components as the enclosure 502 described herein. Enclosure 502 includes a communication module 504 and a modular air quality sensor 506 as described above. In embodiments, the modular air quality sensors are in communication with other components within the structure 501 such that any of these other components can be adjusted based on the air quality sensor data obtained. In embodiments, the modular air quality sensors are configured to transmit sensor data to other systems, such as, home or office automated systems, alarm systems, or building management systems within structure 501. Based on the data provided, the other systems can make adjustments accordingly. For example, if the sensor data indicates unhealthy levels of carbon dioxide or carbon monoxide, a HVAC system can be adjusted so that the area around the sensor is ventilated better.

In embodiments, the air quality sensing system 500 includes cloud-computing system 510 to communicate with the modular air quality sensors. A connection to the internet can be established either directly, through a network, or through any combination thereof. The cloud-computing system 510 can provide one or more services 512 including e.g., software updates, customer support, sensor data collection/logging, remote access, remote or distributed control, or sensor information. Data associated with the services 512 can be stored at the cloud-computing system and the cloud-computing system can retrieve and transmit the data at regular intervals or upon receiving a request.

Sensor data SD from the modular air quality sensors described herein can be received at the services 512 of the cloud-computing system 510. A user can interact with the services 512 through a web browser that is operable on a computer system or mobile device having a wireless data connection with the services 512. The user can also interact with the services 512 through a mobile application. The user interaction through a web browser or mobile application allows the user to retrieve and review the sensor data SD collected by the air quality sensing system and stored within the services 512. The web browser or mobile application can have a graphical user interface that can display information about the sensors that are active in the modular air quality sensor. Such information about the active sensors can be retrieved from the cloud services 512. The graphical user interface can also display readings from each active sensor and such readings can be retrieved from the cloud services 512.

For example, a real-time air quality chart can be displayed for each active sensor on a graphical user interface. In an embodiment including a carbon dioxide sensor, sensor data from a carbon dioxide sensor can be displayed in a format including a line graph showing how the sensor data has changed over a period of time (e.g., the past 24 hours or the past hour or the like). The display can also include a color-coded legend indicating the values that satisfy different thresholds. For example, any eCO2 level between 400 and 1000ppm can be displayed in a first color (e.g., green) to indicate that such levels are a typical acceptable level, any eCO2 level between 1000 and 5000ppm can be displayed in a second color (e.g., yellow) to indicate that such levels are typically accompanied by complaints of health concerns, and any eCO2 level that exceeds 5000ppm can be displayed in a third color (e.g., red) to indicate that such levels are unacceptable for an indoor environment. It should be appreciated that any other suitable thresholds can be used and any other suitable colors can be used. The levels and colors described are not to be construed in a limiting manner. Depending on which level the sensor data satisfies in real-time, the line graph can be displayed in a color corresponding to a color associated with the level the sensor data satisfies. Using the example above, if the eCO2 level is between 400 and 1000ppm, the line graph including the line connecting the data points and the area between the data points and the x-axis can be green or one or more variations of green so that it is immediately apparent that the current level is acceptable despite previous historical levels. Additionally, information about the sensors and a health status for each of the sensors can be displayed by the graphical user interface. The user interaction through a web browser or mobile application can also allow a user to turn on or off a display of each sensor measurement. The user can also initiate an over-the-air (OTA) software update of the modular air quality sensor or program application on the web browser or mobile application.

It should also be understood that, unless clearly indicated to the contrary, in any methods described herein that include more than one step or act, the order of the steps or acts of the method is not necessarily limited to the order in which the steps or acts of the method are recited.

All definitions, as defined and used herein, should be understood to control over dictionary definitions and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

## Claims

1. A dynamically configurable air quality detection system (500), comprising:
an enclosure (10A, 10B, 10C, 502) comprising a communication module (504) and a removable portion (12A, 12B, 12C) to receive one or more air quality sensors (104,120), wherein the enclosure (10A, 10B, 10C, 502) includes at least one of an electrical outlet enclosure, or a switch enclosure;
an integrated circuit board (102) configured to be mounted to or within the enclosure, the integrated circuit board comprising a connector (108) in communication with the communication module;
a first air quality sensor (104) of the one or more air quality sensors (104,120) mounted on the integrated circuit board;
an expansion socket (116) mounted on or arranged within the integrated circuit board, the expansion socket (116) configured to receive an expansion integrated circuit board (118) having a second air quality sensor (120) of the one or more air quality sensors (104,120); and
a microcontroller (106) in communication with the first air quality sensor and the second air quality sensor when the second air quality sensor is installed via the expansion socket;
an array of light emitting elements (112) connected to the integrated circuit board and driven by the microcontroller to indicate a status of the first or second air quality sensors.

2. The system of claim 1, wherein the communication module is configured to transmit sensor status information or sensor data to a building management system.

3. The system of claim 1, wherein the communication module is configured to transmit sensor status information or sensor data to a cloud-computing system.

4. The system of claim 3, further comprising a user interface of a mobile device associated with a user, wherein the user interface is configured to receive sensor status information or sensor data from the cloud-computing system.

5. The system of claim 1, wherein the enclosure is a wall station, a wall receptacle fixture or a ceiling mounted fixture.

6. The system of claim 1, further comprising an onboard radio module within the integrated circuit board, wherein the onboard radio module is configured to transmit sensor status information or sensor data to a cloud-computing system.

7. The system of claim 6, further comprising a user interface of a mobile device associated with a user, wherein the user interface is configured to receive sensor status information or sensor data from the cloud-computing system.

8. The system of claim 1 comprising:
an onboard humidity sensor (S 1), an onboard temperature sensor (S2), and an onboard carbon dioxide sensor (S3) mounted on or within the integrated circuit board;
wherein the expansion socket is configured to receive an expansion integrated circuit board (119) having one or more additional air quality sensors (S4, S5, S6, S7, S8, S9); and
a microcontroller (106) in communication with the onboard humidity sensor, the onboard temperature sensor, and the onboard carbon dioxide sensor, wherein the microcontroller is configured to be in communication with the one or more additional air quality sensors when the one or more additional air quality sensors are installed via the expansion socket;
wherein the array of light emitting elements (112) is configured to indicate a status of the onboard humidity sensor, the onboard temperature sensor, and the onboard carbon dioxide sensor; or
wherein the array of light emitting elements (112) is configured to indicate a status of the one or more additional air quality sensors.

## Patentansprüche

1. Dynamisch konfigurierbares Luftqualitätserkennungssystem (500), umfassend:
ein Gehäuse (10A, 10B, 10C, 502), das ein Kommunikationsmodul (504) und einen abnehmbaren Teil (12A, 12B, 12C) umfasst, um einen oder mehrere Luftqualitätssensoren (104, 120) aufzunehmen, wobei das Gehäuse (10A, 10B, 10C, 502) mindestens eines von einem Stromanschlussgehäuse oder einem Schaltergehäuse einschließt;
eine integrierte Leiterplatte (102), die konfiguriert ist, um an oder in dem Gehäuse montiert zu werden, wobei die integrierte Leiterplatte einen Anschluss (108) umfasst, der mit dem Kommunikationsmodul in Verbindung steht;
einen ersten Luftqualitätssensor (104) des einen oder der mehreren Luftqualitätssensoren (104, 120), der auf der integrierten Leiterplatte montiert ist;
eine Erweiterungsbuchse (116), die auf der integrierten Leiterplatte montiert oder in dieser angeordnet ist, wobei die Erweiterungsbuchse (116) konfiguriert ist, um eine integrierte Erweiterungsleiterplatte (118) mit einem zweiten Luftqualitätssensor (120) des einen oder der mehreren Luftqualitätssensoren (104, 120) aufzunehmen; und
einen Mikrocontroller (106), der mit dem ersten Luftqualitätssensor und dem zweiten Luftqualitätssensor in Verbindung steht, wenn der zweite Luftqualitätssensor über die Erweiterungsbuchse installiert ist;
eine Anordnung von lichtemittierenden Elementen (112), die mit der integrierten Leiterplatte verbunden sind und vom Mikrocontroller angesteuert werden, um einen Status des ersten oder zweiten Luftqualitätssensors anzuzeigen.

2. System nach Anspruch 1, wobei das Kommunikationsmodul konfiguriert ist, um Sensorstatusinformationen oder Sensordaten an ein Gebäudemanagementsystem zu übertragen.

3. System nach Anspruch 1, wobei das Kommunikationsmodul konfiguriert ist, um Sensorstatusinformationen oder Sensordaten an ein Cloud-Computing-System zu übertragen.

4. System nach Anspruch 3, ferner umfassend eine Benutzerschnittstelle einer mit einem Benutzer verknüpften Mobilvorrichtung, wobei die Benutzerschnittstelle konfiguriert ist, um Sensorstatusinformationen oder Sensordaten vom Cloud-Computing-System zu empfangen.

5. System nach Anspruch 1, wobei das Gehäuse eine Wandstation, eine Wandsteckdoseneinrichtung oder eine an der Decke montierte Einrichtung ist.

6. System nach Anspruch 1, ferner umfassend ein bordeigenes Funkmodul innerhalb der integrierten Leiterplatte, wobei das bordeigene Funkmodul konfiguriert ist, um Sensorstatusinformationen oder Sensordaten an ein Cloud-Computing-System zu übertragen.

7. System nach Anspruch 6, ferner umfassend eine Benutzerschnittstelle einer mit einem Benutzer verknüpften Mobilvorrichtung, wobei die Benutzerschnittstelle konfiguriert ist, um Sensorstatusinformationen oder Sensordaten vom Cloud-Computing-System zu empfangen.

8. System nach Anspruch 1, umfassend:
einen bordeigenen Feuchtigkeitssensor (S1), einen bordeigenen Temperatursensor (S2) und einen bordeigenen Kohlendioxidsensor (S3), die auf oder in der integrierten Leiterplatte montiert sind;
wobei die Erweiterungsbuchse konfiguriert ist, um eine integrierte Erweiterungsleiterplatte (119) mit einem oder mehreren zusätzlichen Luftqualitätssensoren (S4, S5, S6, S7, S8, S9) aufzunehmen; und einen Mikrocontroller (106), der mit dem bordeigenen Feuchtigkeitssensor, dem bordeigenen Temperatursensor und dem bordeigenen Kohlendioxidsensor in Verbindung steht, wobei der Mikrocontroller konfiguriert ist, um mit dem einen oder den mehreren zusätzlichen Luftqualitätssensoren in Verbindung zu stehen, wenn der eine oder die mehreren zusätzlichen Luftqualitätssensoren über die Erweiterungsbuchse installiert sind;
wobei die Anordnung von lichtemittierenden Elementen (112) konfiguriert ist, um einen Status des bordeigenen Feuchtigkeitssensors, des bordeigenen Temperatursensors und des bordeigenen Kohlendioxidsensors anzuzeigen; oder
wobei die Anordnung von lichtemittierenden Elementen (112) konfiguriert ist, um einen Status des einen oder der mehreren zusätzlichen Luftqualitätssensoren anzuzeigen.

## Revendications

1. Système de détection de la qualité de l'air pouvant être configuré de façon dynamique (500), comprenant :
un boîtier (10A, 10B, 10C, 502) comprenant un module de communication (504) et une partie amovible (12A, 12B, 12C) destinée à recevoir un ou plusieurs capteurs de qualité de l'air (104, 120), dans lequel le boîtier (10A, 10B, 10C, 502) comporte au moins l'un parmi un boîtier de prise électrique, ou un boîtier de commutateur ;
une carte de circuit intégré (102) configurée pour être montée sur ou dans le boîtier, la carte de circuit intégré comprenant un connecteur (108) en communication avec le module de communication ;
un premier capteur de qualité de l'air (104) parmi le ou les capteurs de qualité de l'air (104, 120) monté sur la carte de circuit intégré ;
un support d'extension (116) monté sur ou disposé dans la carte de circuit intégré, le support d'extension (116) étant configuré pour recevoir une carte de circuit intégré d'extension (118) comportant un second capteur de qualité de l'air (120) parmi le ou les capteurs de qualité de l'air (104, 120) ; et
un microcontrôleur (106) en communication avec le premier capteur de qualité de l'air et le second capteur de qualité de l'air lorsque le second capteur de qualité de l'air est installé par le biais du support d'extension ;
un réseau d'éléments photoémetteurs (112) connectés à la carte de circuit intégré et excités par le microcontrôleur pour indiquer un état des premier ou second capteurs de qualité de l'air.

2. Système selon la revendication 1, dans lequel le module de communication est configuré pour transmettre des informations d'état de capteur ou des données de capteur à un système de gestion de bâtiment.

3. Système selon la revendication 1, dans lequel le module de communication est configuré pour transmettre des informations d'état de capteur ou des données de capteur à un système informatique en nuage.

4. Système selon la revendication 3, comprenant en outre une interface utilisateur d'un dispositif mobile associé à un utilisateur, dans lequel l'interface utilisateur est configurée pour recevoir des informations d'état de capteur ou des données de capteur du système informatique en nuage.

5. Système selon la revendication 1, dans lequel le boîtier est une station murale, un accessoire de logement mural ou un accessoire monté au plafond.

6. Système selon la revendication 1, comprenant en outre un module radio embarqué dans la carte de circuit intégré, dans lequel le module radio embarqué est configuré pour transmettre des informations d'état de capteur ou des données de capteur à un système informatique en nuage.

7. Système selon la revendication 6, comprenant en outre une interface utilisateur d'un dispositif mobile associé à un utilisateur, dans lequel l'interface utilisateur est configurée pour recevoir des informations d'état de capteur ou des données de capteur du système informatique en nuage.

8. Système selon la revendication 1 comprenant :
un capteur d'humidité embarqué (S1), un capteur de température embarqué (S2) et un capteur de dioxyde de carbone embarqué (S3) montés sur ou dans la carte de circuit intégré ;
dans lequel le support d'extension est configuré pour recevoir une carte de circuit intégré d'extension (119) ayant un ou plusieurs capteurs de qualité de l'air supplémentaires (S4, S5, S6, S7, S8, S9) ; et un microcontrôleur (106) en communication avec le capteur d'humidité embarqué, le capteur de température embarqué et le capteur de dioxyde de carbone embarqué, dans lequel le microcontrôleur est configuré pour être en communication avec le ou les capteurs de qualité de l'air supplémentaires lorsque le ou les capteurs de qualité de l'air supplémentaires sont installés par le biais du support d'extension ;
dans lequel le réseau d'éléments photoémetteurs (112) est configuré pour indiquer un état du capteur d'humidité embarqué, du capteur de température embarqué et du capteur de dioxyde de carbone embarqué ; ou
dans lequel le réseau d'éléments photoémetteurs (112) est configuré pour indiquer un état du ou des capteurs de qualité de l'air supplémentaires.
